# EUROPEAN PATENT SPECIFICATION

(11) **EP 4 210 812 B1**
(45) Date of publication and mention of the grant of the patent: **01.04.2026**
(21) Application number: 21867516.3
(22) Date of filing: 08.09.2021
(51) Int. Cl.: A61N 1/04, A61B 5/378, A61B 5/38

(54) **METHODS AND SYSTEMS FOR NEURAL STIMULATION VIA MUSIC AND SYNCHRONIZED RHYTHMIC STIMULATION**
VERFAHREN UND SYSTEME ZUR NERVENSTIMULATION ÜBER MUSIK UND SYNCHRONISIERTE RHYTHMISCHE STIMULATION
PROCÉDÉS ET SYSTÈMES POUR LA STIMULATION NEURONALE PAR LA MUSIQUE ET LA STIMULATION RYTHMIQUE SYNCHRONISÉE

(30) Priority: 08.09.2020 US 202063075516 P
(43) Date of publication of application: 19.07.2023
(73) Proprietor: Oscilloscape, LLC, Farmington, CT 06032 (US)
(72) Inventor: KIM, Ji Chul, Vernon, Connecticut 06066 (US); LARGE, Edward, Willington, Connecticut 06279 (US); LOUI, Psyche, Brookline, Massachusetts 02446 (US)
(74) Representative: Barker Brettell LLP
(86) International application number: PCT/US2021/049470
(87) International publication number: WO 2022/056002

(56) References cited:
- WO-A1-2018/094226
- WO-A1-2019/060298
- WO-A1-2020/056418
- US-A1- 2019 247 662
- US-A1- 2019 314 641
- US-A1- 2019 388 020
- US-A1- 2020 164 201

## Description

### CROSS REFERENCE TO RELATED APPLICATIONS

The present invention derives priority from U.S. Provisional Patent Application No. 63/075,516, filed September 8, 2020.

### BACKGROUND OF THE INVENTION

### Field of the Invention

The present invention relates generally to methods and systems for neural stimulation. In particular, the methods and systems of the present disclosure can provide stimulation signals, including musical and visual stimulation signals, to induce synchronized oscillations in the brain of a subject.

### Description of the Background

Neural oscillation occurs in humans and animals and includes rhythmic or repetitive neural activity in the central nervous system. Neural tissue can generate oscillatory activity by mechanisms within individual neurons or by interactions between neurons. Oscillations can appear as either periodic fluctuations in membrane potential or as rhythmic patterns of action potentials, which can produce oscillatory activation of post-synaptic neurons. Synchronized activity of a group of neurons can give rise to macroscopic oscillations, which can be observed by sensing electrical or magnetic fields in the brain using techniques such as electroencephalography (EEG), intracranial EEG (iEEG), also known as electrocorticography (ECoG), and magnetoencephalography (MEG).

Neural oscillations can be characterized by their frequency, amplitude, and phase. These signal properties can be observed from neural recordings using time-frequency analyses. For example, an EEG can measure oscillatory activity among a group of neurons, and the measured oscillatory activity can be categorized into frequency bands as follows: delta activity corresponds to a frequency band from 0.5 - 4 Hz; theta activity corresponds to a frequency band from 4-8 Hz; alpha activity corresponds to a frequency band from 8-13 Hz; beta activity corresponds to a frequency band from 13-30 Hz; and gamma activity corresponds to a frequency band of 30 Hz and above.

Neural oscillations of different frequency bands can be associated with cognitive states or cognitive functions such as perception, action, attention, reward, learning, and memory. Based on the cognitive state or cognitive function, the neural oscillations in one or more frequency bands may be involved. Further, neural oscillations in one or more frequency bands can have beneficial effects or adverse consequences on one or more cognitive states or functions.

Neural entrainment occurs when an external stimulation of a particular frequency or combination of frequencies is perceived by the brain and triggers neural activity in the brain that results in neurons oscillating at frequencies related to the particular frequencies of the external stimulation. Thus, neural entrainment can refer to synchronizing neural oscillations in the brain using external stimulation such that the neural oscillations occur at the frequencies corresponding to the particular frequencies of the external stimulation. Neural entrainment can also refer to synchronizing neural oscillations in the brain using external stimulation such that the neural oscillations occur at frequencies that correspond to harmonics, subharmonics, integer ratios, and combinations of the particular frequencies of the external stimulation. The specific neural oscillatory frequencies that can be observed in response to a set of external stimulation frequencies are predicted by models of neural oscillation and neural entrainment.

Cognitive functions such as learning and memory involve coordinated activity across distributed subcortical and cortical brain regions, including hippocampus, cortical and subcortical association areas, sensory regions, and prefrontal cortex. Across different brain regions, behaviorally relevant information is encoded, maintained, and retrieved through transient increases in the power of and synchronization between neural oscillations that reflect multiple frequencies of activity.

In particular, oscillatory neural activity in the theta and gamma frequency bands are associated with encoding, maintenance, and retrieval processes during short-term, working, and long-term memory. Induced gamma activity has been implicated in working memory, with increases in scalp-recorded and intracranial gamma-band activity occurring during working-memory maintenance. Increases in the power of gamma activity dynamically track the number of items maintained in working memory. Using electrocorticography (ECoG), one study found enhancements in gamma power tracked working-memory load in the hippocampus and medial temporal lobe, as participants maintained sequences of letters or faces in working memory. Finally, other evidence indicates that hippocampal gamma activity aids episodic memory, with distinct sub-gamma frequency bands corresponding to encoding and retrieval stages.

Theta oscillations (4 - 8 Hz) have been linked to working and episodic memory processes. Intracranial EEG (iEEG) recordings demonstrate that, during working memory, theta oscillations gate on and off (i.e., increase and sustain in amplitude, before rapidly decreasing in amplitude) over the encoding, maintenance, and retrieval stages. Other work has observed increases in scalp-recorded theta activity during working-memory maintenance. A recent systematic review concluded that scalp-recorded theta activity, emerging from frontal-midline electrodes, was the most robust neural correlation of verbal working-memory maintenance. Moreover, frontal-midline theta activity tracks working-memory load, increasing and sustaining in power as a function of the number of items maintained in working memory.

Prior art studies have found that gamma-frequency, auditory-visual stimulation can ameliorate dementia or Alzheimer's Disease (AD)-related biomarkers and pathophysiologies, and, if administered during an early stage of disease progression, can provide neuroprotection.

However, auditory stimulation at gamma frequencies is perceived as rough and unpleasant to human listeners. In a prior art study by Malchano & Williams, the subject's physiological and attentional states are monitored, while "orchestration policies" switch between modalities of stimulation within treatment sessions. This study found that gamma frequency auditory stimulation may not be well-tolerated by patients, and may be problematic to administer for the long periods of time required to produce clinical benefits.

To combat the rough and unpleasant nature of auditory stimulation at gamma frequencies, prior art solutions have contemplated embedding gamma frequency auditory stimulation within musical signals - reasoning that a spoonful of sugar may help the medicine go down. There are several problems with this approach. First, most music includes changing bass frequencies in the gamma range, and bass frequencies are the most important cue to harmony perception. Thus, it may be difficult or impossible to incorporate additional gamma frequency sounds in a manner that is harmonious with the existing music. In other words, this approach would not cover up the aversive auditory gamma stimulation; it would simply make the music sound bad.

Yet another problem with embedding gamma frequency auditory stimulation within a musical signal is that the brain's natural response to music already includes gamma frequency responses. The natural neural gamma frequency response to music can interfere with additional auditory gamma stimulation in a way that renders the therapeutic stimulation ineffective. Additionally, prior art systems only stimulate one specific frequency. A common outcome of such stimulation is neural adaptation, which leads to a reduced neural response over time.

U.S. Patents No. 10,293,177, No. 10,279,192, and No. 10,307,611, all assigned to Cognito Therapeutics, Inc., are examples of this type of prior art gamma frequency auditory stimulation. The technology disclosed therein includes methods for playing musical audio signals in addition to auditory stimulus pulses at a specific gamma frequency. As noted above, however, the pure overlay of gamma frequency audio stimulus onto an existing audio signal decreases the patient's ability to tolerate the treatment and lowers the efficacy of the treatment. WO2018/90094226 describes systems and methods directed to neural stimulation using visual and auditory stimuli.

### BRIEF SUMMARY OF THE INVENTION

To solve these and other prior art problems, in preferred embodiments, systems and methods of the present disclosure are directed to neural stimulation via rhythmic light stimulation that is presented simultaneously with auditory stimulation through music. The combination of music and light stimuli can elicit neural oscillation effects or stimulation. The combined stimuli can adjust, control or otherwise affect the frequency of the neural oscillations to provide beneficial effects to one or more cognitive states, cognitive functions, the immune system or inflammation, while mitigating or preventing adverse consequences on a cognitive state or cognitive function. For example, systems and methods of the present technology can treat, prevent, protect against or otherwise affect Alzheimer's Disease.

### BRIEF DESCRIPTION OF THE DRAWINGS

In the figures:
Fig. 1 provides a breakdown of the frequencies selected by the OSM in one exemplary embodiment as they relate to a specific underlying musical stimulus, and the range of frequencies present in each frequency band, in preferred embodiments of the present invention.
Fig. 2 shows, on the left hand side, MEG recordings of human auditory cortex recorded while subjects listened to rhythmic auditory stimuli at two different tempos, and on the right hand side, highlights of some of the brain areas that exhibited this response.
Fig. 3 is a schematic diagram showing the various components / modules of the inventive system, in an embodiment of the present invention.
Fig. 4 is a schematic diagram showing the high-level interoperability of the inventive components coupled with the resultant brain stimuli, in an embodiment of the present invention.
Fig. 5 illustrates a stimulus provided by one song in an embodiment of the present invention. Panel A compares the auditory rhythmic frequencies (i.e., the onset spectrum) of the music with the frequency of an auditory 40 Hz pulse train. Panel B compares the visual frequencies stimulated by the present invention with the frequency of a visual 40 Hz pulse train.
Fig. 6 illustrates a stimulus provided by a second song in an embodiment of the present invention. Panel A compares the auditory rhythmic frequencies (i.e., the onset spectrum) of the music with the frequency of an auditory 40 Hz pulse train. Panel B compares the visual frequencies stimulated by the present invention with the frequency of a visual 40 Hz pulse train.
Fig. 7 is a diagram of a device for delivering visual stimulation, in an embodiment of the present invention.

### DETAILED DESCRIPTION OF THE PREFERRED EMBODIMENTS

The present inventors have discovered that music entrains and drives neural activity in multiple frequency ranges, and that therefore musical stimulation itself can entrain and drive oscillatory neural activity that is involved in learning, memory, and cognition. In preferred embodiments, the present invention relies on the brain's natural delta, theta, and gamma frequency responses to music by providing music as the sole auditory stimulus in a system and method for treating, preventing, protecting against or otherwise affecting Alzheimer's Disease and dementia. In preferred embodiments of the invention, the audio stimulus is coupled with visual stimulation in the delta, theta, and/or gamma frequency bands, which is choreographed by an entrainment simulator to synchronize with the delta, theta and/or gamma frequency bands of the brain's response to the audio stimulus for enhanced therapeutic effect. In other embodiments of the present invention, additional frequencies and frequency bands can be targeted for stimulation, to treat, prevent, and/or protect against Alzheimer's Disease, dementia, and/or other ailments such as Parkinson's Disease.

It has been observed by the present inventors that musical rhythms are organized into well-structured frequency combinations. For example, musical rhythms entrain neural activity in the delta and theta frequency ranges by directly stimulating the brain at these frequencies. The frequency of the basic beat typically corresponds to neural activity in the delta frequency band. Subdivisions of the beat typically correspond to neural activity in the theta frequency band. It has also been found that musical rhythms can drive activity at delta and theta frequencies that are not explicitly present in the rhythms, because musical rhythms contain structured frequency combinations. Frequencies observed in brain activity can include harmonics, subharmonics, integer ratios, and combinations of frequencies present in the musical rhythms, and are predicted by simulations of neural oscillation and neural entrainment.

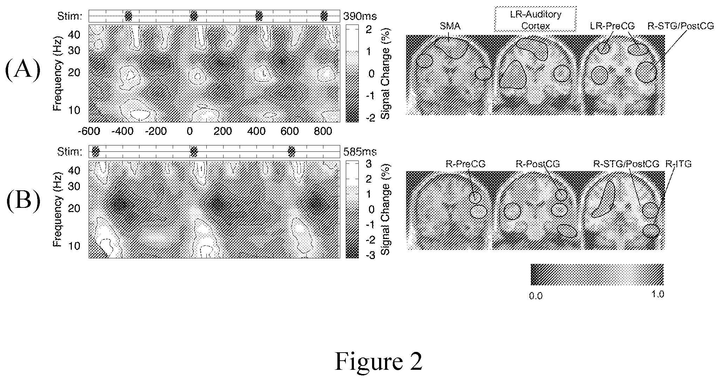

The inventors have also observed that musical rhythms can drive gamma neural activity in the brain in a way that is different than the entrainment of delta and theta activity. The amplitude of endogenous gamma neural oscillations is modulated, such that amplitude peaks synchronize with musical events (see Fig. 2). Amplitude modulation of gamma neural activity reflects phase-amplitude coupling to lower frequency (e.g., delta and theta) neural activity. Phase-amplitude coupling (PAC) is defined as a statistical dependency between the amplitude of oscillations in one frequency band and the phase of oscillations in another frequency band. For example, in theta-gamma phase-amplitude coupling, peaks in gamma amplitude correspond to a specific phase of entrained theta activity. Thus, gamma activity is driven by entrained theta and delta activity.

The inventive system and corresponding methods work by activating the brain's natural delta, theta, and gamma responses to music in a way that does not interfere with musical enjoyment. Because enjoyment is critical for patient tolerability and completion of protocols, the present invention overcomes the drawbacks of the prior art methods which de-incentivize patient compliance with the treatment by including the abrasive and unpleasant sounds of added audio waves in the gamma frequency band.

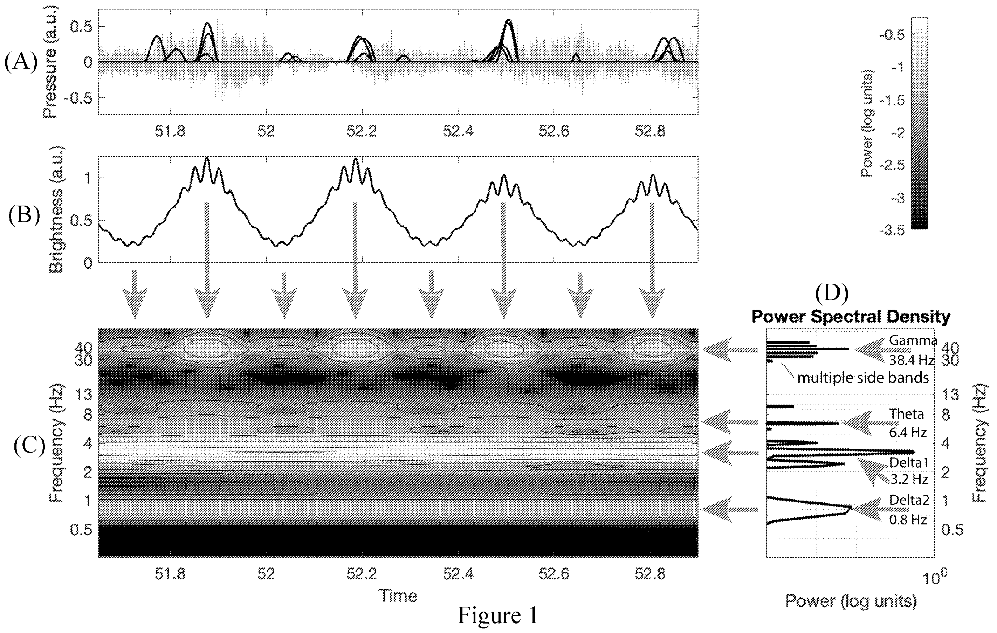

In preferred embodiments, the invention also employs visual stimulation in the delta, theta, and/or gamma frequency bands, so as to enhance (as opposed to conflicting with, as in the case of the prior art) the frequencies that are important in musical enjoyment. The inventors believe that this additional aspect of the invention enhances the efficacy of the inventive method because visual stimulation in the gamma band is less aversive than auditory stimulation in the gamma band. In some embodiments, gamma stimulation can be combined with delta and theta stimulation to create visual stimulation that mimics the brain's natural response to musical rhythms. In the inventive system and method, gamma stimulation can be amplitude-modulated (see Fig. 1) through phase-amplitude coupling to theta and/or delta frequency oscillations to mimic auditory processing, increasing the efficacy and extent of neural stimulation. Furthermore, the specific stimulus frequencies are determined by the musical stimuli, and so stimulus frequencies provided by the present invention change within a stimulus session, decreasing the potential for neural adaptation, and thus increasing stimulus efficacy. In these embodiments, the invention thus combines music listening with delta, theta, and/or gamma frequency visual stimulation to create engaging, and effective audiovisual stimuli for patients.

In other embodiments, as noted earlier, additional frequency bands may be employed, both via audio or visual stimuli.

In preferred embodiments, the inventive solution outputs an improved set of stimuli which amplify the brain's natural delta, theta, and gamma responses to music in a way that does not create neural interference between the brain's natural oscillatory responses to music and added oscillatory auditory stimulation within the same frequency bands. Specifically, in certain embodiments, the inventive system and method uses a simulation of neural entrainment to determine the frequencies of the brain's natural delta, theta, and gamma responses to music. The inventive system then reinforces and amplifies the natural responses to music by delivering the same delta, theta, and/or gamma frequencies in visual stimulation. The simulation can include delta-theta-gamma phase-amplitude coupling to faithfully mimic the brain's auditory response, and amplify the effect. Thus, the visual stimulation does not interfere with, or cancel, the brain's natural oscillatory responses to music (as in prior art methods), but amplifies them.

Systems and methods of the present invention are directed to outputting stimuli which elicit neural stimulation via rhythmic light stimulation that is presented simultaneously with musical stimulation. The inventive combination of music and rhythmic light pulses can elicit brainwave effects or stimulation. The combined stimuli can adjust, control or otherwise affect the frequency of the neural oscillations to provide beneficial effects to one or more cognitive states, cognitive functions, the immune system or inflammation (or other conditions), while mitigating or preventing adverse consequences on a cognitive state or cognitive function, and maximizing enjoyment, treatment tolerability, and completion of treatment protocol. For example, systems and methods of the present technology can treat, prevent, protect against or otherwise affect Alzheimer's Disease.

The frequencies of neural oscillations observed in patients can be affected by or correspond to the frequencies of the musical rhythm and the rhythmic light pulses. Thus, systems and methods of the present invention can elicit neural entrainment by outputting multi-modal stimuli such as musical rhythms and light pulses emitted at frequencies determined by analysis of the musical rhythm. This combined, multi-modal stimulus can synchronize electrical activity among groups of neurons based on the frequency or frequencies that are entrained and driven by musical rhythm. Neural entrainment can be observed based on the aggregate frequency of oscillations produced by the synchronous electrical activity in ensembles of neurons throughout the brain.

In other embodiments, additional outputs from the system may also include one or more stimulation units for generating tactile, vibratory, thermal and/or electrical transcutaneous stimuli. Such stimulation units may include a mobile device, smart watch, gloves, or other devices that can vibrate. In other embodiments the output device may include stimulation units for generating electromagnetic fields or electrical currents, such as an array of electromagnets or electrodes, to deliver transcranial stimulation.

To achieve the inventive method, the system according to the present invention includes an Auditory Analysis System (AAS) comprising means to receive an auditory input, filter the acoustic signal, detect the onset of acoustic events (e.g., notes or drum hits) and adjust the gain of the resulting signal. In embodiments, the primary function of the AAS is preprocessing an auditory stimulus to provide multi-channel rhythmic inputs (e.g., note onsets). In some embodiments of the present invention, the auditory input is provided by the system, such as by a built-in audio playback system that has access to a library of songs and/or other musical compositions. In such embodiments, the system may further comprise a graphical display and input means accessible to the user (e.g. patient or therapist) to allow the user to make a selection from the library for playback. In other embodiments, in addition to or as an alternative to a built-in audio playback system, the inventive system comprises an auxiliary audio input to allow the system to receive input from a secondary playback system, such as a personal music playback device (e.g. an iPod, MP3 player, smart phone, or the like). In yet other embodiments, in addition to or as an alternative to the above auditory input means, the system comprises a microphone or like means to allow the system to receive auditory input from ambient sound, such as a live musical performance or music broadcast from secondary speakers, such as the user's home stereo system. In order to facilitate one or more of the above auditory input means, in some embodiments, the AAS interfaces with a profile manager comprising a processor or internet-enabled software application accessing non-transitory and/or random-access memory which stores data pertaining to one or more users or patients, such as identifying information (e.g. name or patient ID number) stored information from previous therapies, and/or a library of audio files, in addition to various user preferences, such as song selection.

In embodiments where the audio signal is received by the system through a built-in playback system or auxiliary input such as through a MP3 player, the system may further comprise headphones or integrated speakers to allow the listener to hear the audio signal in real time.

As noted above, the AAS further comprises a filtering module, an onset detection module, and an optional gain control module to filter the signal, detect the onset of acoustic events, and adjust a gain of the resulting signal, respectively.

In some preferred embodiments, the system of the present invention utilizes an Entrainment Simulator (ES) which receives and processes the received audio signal(s) to simulate processing in the human brain, in order to suggest and output oscillation signals to enhance the received audio signal(s) and thereby enhance the therapeutic effect of the inventive treatment. In some embodiments, the AAS is operatively connected to the ES and provides data to the ES in the form of an onset signal. In some embodiments, the ES also interfaces with the profile manager to, e.g., recall patient data from prior therapies. In some embodiments, the primary function of the ES is to simulate entrained neural oscillations to predict the frequency, phase, and amplitude of the human neural response to music.

Also in some preferred embodiments, the inventive system includes an Oscillation Selection Module (OSM). In embodiments, the OSM receives input from the ES and outputs one or more selected oscillation states as frequencies, amplitude, and phases, for visual stimulation.

Collectively, the previously-described components enable the inventive system to, in preferred embodiments, (1) receive auditory input through the one or more means for receiving an audio signal described above, (2) simulate neural entrainment to the pre-processed auditory signal using one or more Entrainment Simulator(s), which may include multi-frequency artificial neural oscillator networks, (3) couple oscillations within the networks using phase-amplitude or phase-phase coupling, (4) use adaptive learning algorithms to adjust coupling parameters and/or intrinsic parameters, and/or (5) select the most prominent oscillations in one or more frequency bands for display as a visual stimulus, via the Brain Rhythm Stimulator, described below.

As noted, in some embodiments, the ES comprises one or more oscillatory neural networks designed to simulate neural entrainment. In embodiments, an artificial oscillatory neural network receives a preprocessed an auditory stimulus (music), and entrains simulated neural oscillations to predict the frequency, phase, and relative amplitudes of the human neural response to the music. The OSM then selects the most prominent oscillations in one or more predetermined frequency ranges (in preferred embodiments, the delta, theta, and gamma frequency bands) for visual stimulation. Via the Brain Rhythm Stimulator (described below), the simulated neural oscillations are used to synchronize visual stimulation in the selected frequency ranges to the rhythm of music via a device such as an LED light ring, as described below.

In some embodiments, the ES may comprise any other means of mimicking an oscillatory neural network, including but not limited to a deep neural network, an oscillator network, a set of numerical formulae, an algorithm, or any other sufficient means. For these purposes, sufficiency means that the simulator should be able to correctly predict the frequencies, phases, and relative amplitudes of oscillations in the typical human brain that are entrained and driven by any given musical stimulus. It should be capable of correctly predicting the responses in at least the delta (1-4 Hz), theta (4-8 Hz) and low gamma (30-50 Hz) frequency bands.

In some embodiments, the OSM according to the present invention couples the visual gamma frequency stimulation to the beat and rhythmic structure of music through phase-amplitude coupling (as described by, e.g., Lakatos et al, An Oscillatory Hierarchy Controlling Neuronal Excitability and Stimulus Processing in the AuditoryCortex, J Neurophysiol 94:1904-1911, 2005. First published 18 May 2005, the contents of which are incorporated herein by reference in their entirety). In preferred embodiments, the OSM selects variable, music-based frequencies in the delta, theta and gamma ranges for visual stimulation to the user, which stimulation is produced by the Brain Rhythm Stimulator (BRS) as described below. Fig. 1 provides a breakdown of four frequencies selected by the OSM in one exemplary embodiment as they relate to the underlying music, and the range of frequencies present. In some embodiments, the OSM selects one or more harmonically related frequencies in the delta, theta, and lower gamma (30-50 Hz) frequency ranges. In some embodiments, the gamma amplitude is modulated by the theta frequency, simulating theta-gamma phase-amplitude coupling. Also in some embodiments, the theta amplitude is modulated by one or more delta frequencies, simulating the delta-theta phase amplitude coupling. Collectively, the foregoing, thereby simulates the delta-theta-gamma oscillatory hierarchy in the auditory cortex.

With continued reference to Fig. 1, there is illustrated an exemplary protocol for visual stimulation frequencies produced by the inventive system in the gamma, theta and delta frequency bands according to an aspect of the present invention. Panel A shows the time-domain waveform of the music stimulus over a 4 beat time interval, and the onsets computed during preprocessing. Panel B shows the delta-theta-gamma coupled changes in brightness provided by the OSM, while Panel C shows the same changes in each frequency band. By comparison, Fig. 2 shows an MEG recording of a human auditory cortex recorded while the subject listened to two rhythms with different tempos. Panel A of Fig. 2 is a time-frequency map of signal power changes related to rhythmic stimuli presented every 390 ms (2.6Hz), which shows a periodic pattern of signal increases and decreases in the gamma frequency band. Panel B shows the same measurement with respect to a rhythmic stimuli presented every 585 ms (1.7Hz). In the auditory cortex, gamma is amplitude modulated by delta and theta, and this pattern is simulated by the inventive device. Panel D of Fig. 1 illustrates the stimulus produced by the inventive device in the frequency domain. Collectively, these figures illustrate that gamma oscillations are effectively stimulated by the output provided by the inventive device in a range of frequencies around the main frequency. These additional frequencies are called sidebands, and they are caused by the inventive device and method's amplitude modulation from theta and delta frequencies. Moreover, each song played by the inventive device leads to a different choice of frequencies within the delta, theta, and gamma ranges. Thus, over the course of several songs played via the inventive device, the output provided by the inventive device stimulates many gamma frequencies. By contrast, prior art devices only stimulate a single gamma frequency.

The device thus simulates an amplitude modulation of the stimulus provided in the gamma frequency band by the phase of stimulation provided in the delta and theta frequency bands, which mimics the brain's natural gamma-delta-theta phase-amplitude coupling response and thereby enhances both tolerance and efficacy of the treatment. As noted above, Panel D of Fig. 1 shows that gamma oscillations are effectively stimulated in a range of frequencies (sidebands) around the main frequency. These sidebands are caused by the amplitude modulation from theta and delta frequencies provided by the inventive system.

Moreover, each musical composition played by the system leads to a different choice of frequencies within the delta, theta, and gamma ranges. Thus, over the course of one session, different gamma frequencies are stimulated. By contrast, prior art systems only stimulate one frequency, and a common outcome is neural adaptation, leading to a reduced neural response. In preferred embodiments of the present system, changing frequencies avoid neural adaptation and promote robust neural responses.

In embodiments, the rhythmic visual stimulus selected for output to the user (as described below) therefore includes delta, theta, and/or gamma frequencies, as well as theta-gamma and/or delta-gamma phase-amplitude coupling to enhance naturally occurring oscillatory responses to musical rhythm. The sensory cortices (e.g. primary visual and primary auditory cortices) in the brain are functionally connected to areas important for learning and memory, such as the hippocampus and the medial and lateral prefrontal cortices. Thus, coupling a complex rhythmic visual stimulus, including delta, theta, and gamma-frequency visual stimulation to musical rhythm can drive theta, gamma, and theta gamma coupling in the brain, activating neural circuitry involved in learning, memory, and cognition. This, in turn, can drive learning and memory circuits involved in music. In some embodiments, the system incorporates means to prompt a user to choose his or her own individualized music preferences as an auditory stimulus, which can maximize effectiveness of the given system by stimulating auditory and reward systems in patients with early stages of dementia and cognitive decline.

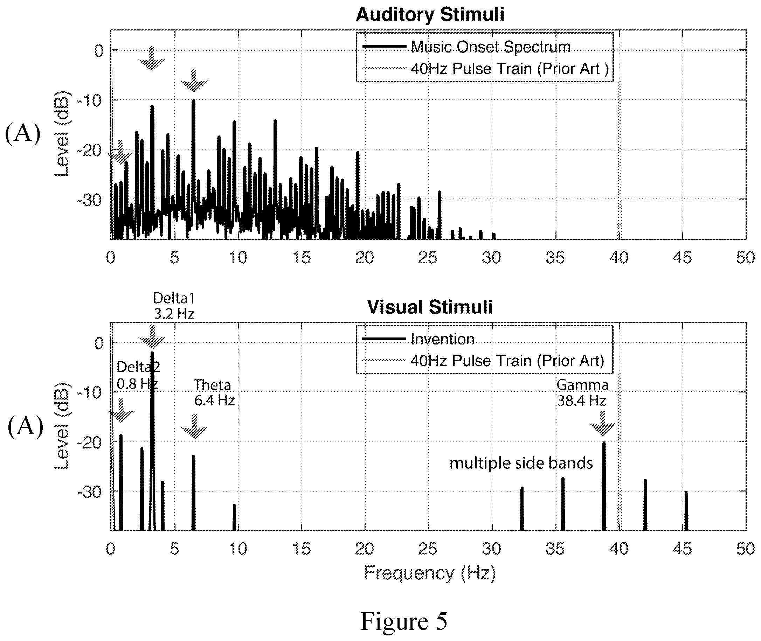

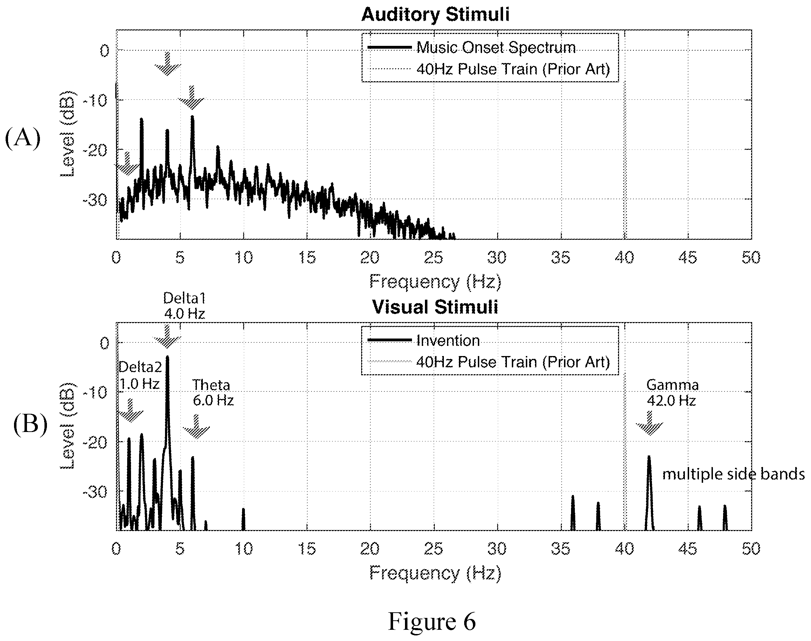

Comparisons between the auditory and visual stimulus provided by the present invention as compared with an exemplary prior art device are shown in Figs. 5 and 6. Both figures illustrate the diverse frequencies of audio and visual stimuli provided by both the instant invention and the prior art device. Fig. 5 and Fig. 6 each illustrate a stimulus provided by a different song. As can be seen, the prior art device provides both audio and visual stimulation at a single frequency, which can easily be contrasted with the broad range of frequencies at which the instant inventive device provides both audio and visual stimulation.

In preferred embodiments, rhythmic visual stimulation is output to the user via a Brain Rhythm Stimulator (BRS) which is operatively connected to the ES and optional OSM within the inventive system. The BRS can include a pattern buffer, a generation module, adjustment module, and a filtering component, and may be operatively connected to an output device comprising a means of displaying rhythmic light stimulation. The output device may include LED lights, a computer monitor, a TV monitor, goggles, virtual reality headsets, augmented reality glasses, smart glasses, or other suitable stimulation output devices. In some embodiments the output device may be a stimulation unit for generating tactile, vibratory, thermal and/or electrical transcutaneous stimuli, such as in a wearable device, smart watch, or mobile device. In some embodiments the output device may include a stimulation unit for generating electromagnetic fields or electrical currents, such as an array of electromagnets or electrodes, to deliver transcranial stimulation. The BRS can also interface with the profile manager which, as noted above, comprises a processor or internet-enabled software application accessing non-transitory and/or random-access memory which stores data pertaining to one or more users or patients. Thus, in some preferred embodiments, information stored by the profile manager may also include previously-captured or user-selected preferences of patterns, waveforms or other parameters of stimulation, such as colors, preferred by the user/patient.

Collectively, these components enable the BRS to (1) read the patient's profile from the profile manager, (2) select a pattern based on the profile, (3) retrieve one or more selected oscillatory signals and/or states from the ES/OSM, (4) generate a pattern, (5) adjust the pattern based on the profile, and (5) display or output the rhythmic stimulation on an output device. In some embodiments, a pattern refers to a light pattern, and an output device refers to a visual output device.

In some embodiments, a visual output device according to the present invention can include integrated or connected LED lights, a computer monitor, a TV monitor, goggles, virtual reality headsets, augmented reality glasses, smart glasses, or other suitable light output device. In some embodiments the output device may be a stimulation unit for generating tactile, vibratory, thermal and/or electrical transcutaneous stimuli, such as in gloves, a smart watch or other wearable device, or a mobile device. In some embodiments the output device may a stimulation unit for generating electromagnetic fields or electrical currents, such as an array of electromagnets or electrodes, to deliver transcranial stimulation.

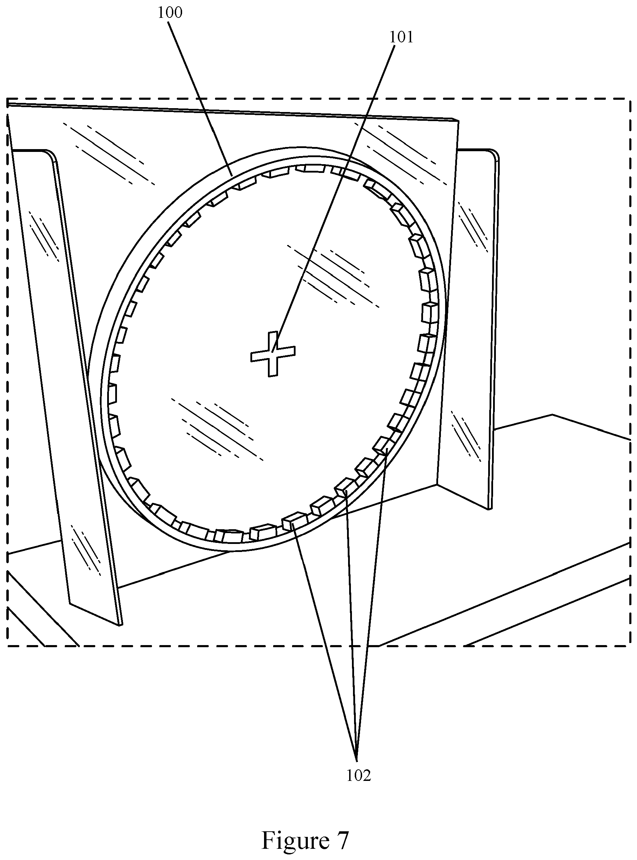

In one preferred embodiment, with reference to Fig. 7, visual stimulation is provided via a visual stimulation ring 100 comprising LED lights 102 that are operatively connected to the remaining components of inventive device and/or to the BRS component. In some embodiments, the visual stimulation ring 100 is positioned in front of the participant, who is asked to foveate on the center, indicated by reference character 101. In some embodiments, the visual stimulation ring 100 is placed at the appropriate distance to stimulate the retina at a specific visual angle. For example, the ring may be placed at the appropriate distance to stimulate the retina at a visual angle of between 0 and 15 degrees, or between 10 and 60 degrees, or between 15 and 50 degrees, or between 15 and 25 degrees, or between 18 and 22 degrees, or between 19 and 21 degrees. In some embodiments, the visual stimulation ring 100 may be placed at the appropriate distance to stimulate the retina at a visual angle of 20 degrees where the maximum density of rods is found in the retina.

Finally, in preferred embodiments, the inventive system includes a Brain Oscillation Monitor (BOM) which provides neural feedback that can be used to optimize the frequency, amplitude, and phase of the visually presented oscillations so as to optimize the frequency, phase, and amplitude of the oscillations in the brain. In embodiments, the BOM provides feedback to the system to allow it to adjust parameters to optimize the phase of outgoing oscillation signals. The BOM can include, interface with, or otherwise communicate with electrodes, magnetometers, or other means of sensing brain activity, a signal amplifier, a filtering component, and a feedback interface component. In preferred embodiments, feedback is provided in the form of EEG signals to the ES.

Collectively, these components enable the BOM to identify the frequency, phase, and amplitude of brain oscillations entrained by the stimulus. The brain oscillation monitor senses electrical or magnetic fields in the brain, amplifies the brain signal, filters the signal to identify specific neural frequencies, and provides input to the ES as set forth above. Means for the BOM to sense electrical or magnetic fields in the brain can include electrodes connected to an electroencephalogram (EEG), intracranial EEG (iEEG), also known as electrocorticography (ECoG), magnetoencephalography (MEG), and other means known in the art.

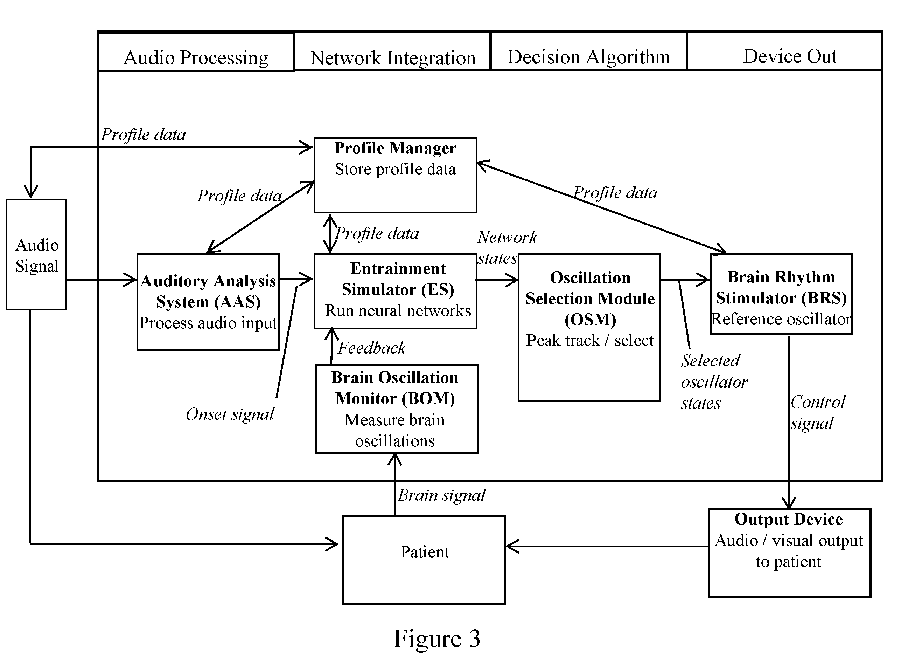

A schematic diagram showing the various components / modules of the inventive system is shown in Fig. 3.

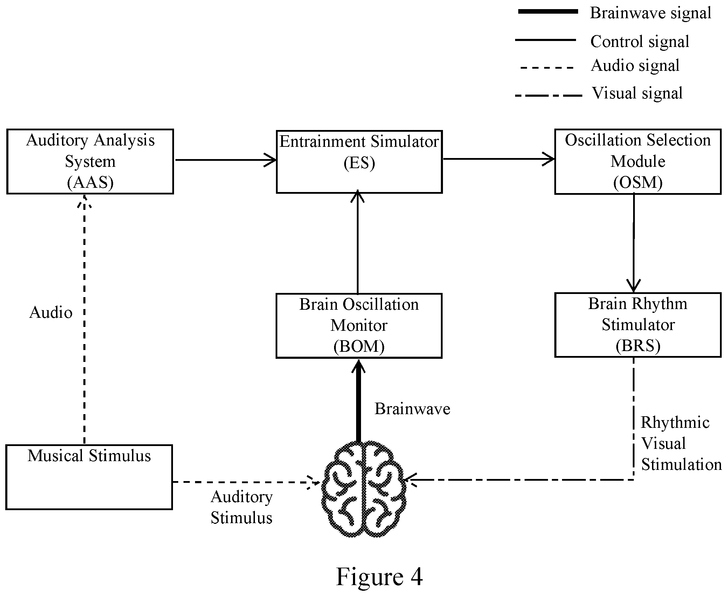

In some embodiments, a schematic diagram showing the high-level interoperability of the inventive components coupled with the resultant brain stimuli is shown in Fig. 4.

Accordingly, in a method according to one embodiment of the present invention, the system:
(A) receives an auditory input,
(B) filters the acoustic signal,
(C) detects the onset of acoustic events,
(D) simulates neural entrainment to the pre-processed auditory signal using one or more multi-frequency neural oscillator networks,
(E) couples oscillations within the networks using phase-amplitude or phase-phase coupling,
(F) uses adaptive learning algorithms to adjust coupling parameters and/or intrinsic parameters,
(G) select the most prominent oscillations in the delta, theta, and/or gamma frequency bands for display,
(H) generates a light pattern, and
(I) displays the rhythmic light on a visual output device.

In some preferred embodiments, prior to receiving an audio input the system also performs the steps of prompting the user to select a source of audio input and/or to make a selection from a library of songs or musical compositions stored by the system.

In particular, the present inventors have discovered that self-selected music, that is, music that an individual patient has selected and which he/she is familiar with, is more effective at engaging larger networks of brain activity compared to music selected by others, or music that the patient is not familiar with, in regions of the brain that include the hippocampus as well as the auditory cortex and the frontal lobe regions that are important for long-term memory. They have also found that listening to familiar music is more effective at driving brain activity in older adults, and it activates more brain areas. Importantly, familiar music drives greater activation in the hippocampus, a key region for memory.

Data developed from the present inventors show that music that is selected by the listener is much more likely to be well-liked and familiar to the listener, and is much more effective at engaging brain activity than music that is selected by researchers. In particular, the present inventors have found that, with respect to the present invention, self-selected music increases activity in the dopaminergic reward system, in the default mode network, and in predictive processes of the brain, in addition to activating the auditory system. Without being bound by any particular theory, the present inventors have observed that that prolonged music listening increases the functional connectivity of the brain from sensory cortices towards the dopaminergic reward system, which is responsible for a variety of motivated behaviors.

Therefore, in preferred embodiments of the present invention, the auditory stimulus comprises music which is self-selected by patients, which has the practical impact of maximizing engagement throughout the brain. The present invention thus empowers patients to listen to their favorite musical recordings while watching captivating, audiovisual displays that include delta, theta gamma-frequency stimulation, further improving patient compliance with the disclosed treatment protocol(s).

In yet other preferred embodiments, prior to the steps of generating and displaying a light pattern, the system further prompts the user to select a profile from an input device and/or user interface integrated in or coupled with the system, and performs the following steps: (G2) reads the patient's profile from the profile manager, (G3) selects a light pattern based on the profile, (G4) retrieves one or more oscillatory signals from the ES, (H) generates a light pattern, and (H2) adjusts the light pattern based on the profile.

In certain preferred embodiments, the system also optimizes the frequency, phase, and/or amplitude of outgoing oscillation signals based on data received from the BOM. Accordingly, the system, on an intermittent or ongoing basis, performs the following additional steps: (J) receives input from the BOM, (K) provides input to the ES, (L) couples input through phase-phase coupling, and (M) uses adaptive learning algorithms to adjust coupling parameters and/or intrinsic parameters to optimize the frequency, phase, and amplitude of outgoing oscillation signals.

Thus, the methods according to the present invention provide neural stimulation to a user via at least a presentation of rhythmic visual stimulation simultaneously, synchronously and in coordination with, musical stimulation.

For example, in other embodiments of the present invention, the system generates and displays light patterns based on system self-selection or on profile data housed for an individual user to be displayed simultaneously with musical stimulation. In such embodiments, the system:
(A) selects one or more oscillations in the delta, theta, and/or gamma frequency bands,
(B) generates a light pattern using the one or more oscillations selected, and
(C) displays said light pattern on the visual output device.

The system in this embodiment may also consult a user's profile and selects a light pattern based on the profile. The system may first prompt the user to select a profile from an input device and/or user interface integrated in or coupled with the system, and read the patient's profile from the profile manager in order to determine the proper light pattern to display.

As described herein with respect to various embodiments, the auditory analysis system receives auditory input through a microphone or auxiliary audio input, filters the acoustic signal, detects onset of acoustic events (e.g., notes or drum hits), and adjusts the gain of the resulting signal.

As described herein with respect to various embodiments, the ES receives auditory input from auditory analysis system, simulates neural entrainment to the pre-processed auditory signal using one or more multi-frequency neural oscillator networks using said input, couples oscillations within the networks using phase-amplitude or phase-phase coupling, uses adaptive learning algorithms to adjust coupling parameters and/or intrinsic parameters, selects oscillations for display in the predetermined frequency ranges, based on a retrieved profile. It also receives input from the brain oscillation monitor, provides input to one or more multi-frequency neural networks, couples neural input through phase-phase coupling, and uses adaptive learning algorithms to adjust coupling parameters to optimize the amplitude and phase of outgoing oscillation signals.

As described herein with respect to various embodiments, the BRS reads the patient's profile from the profile manager, selects a light pattern based on the profile, reads one or more oscillatory signals from the ES, selects at least one of a delta frequency, a theta frequency, a gamma frequency, and or a combination of frequencies, whose frequencies, amplitudes and phases are determined by the entrainment simulator, generates a rhythmic light pattern based on the selected frequencies, adjusts the light pattern based on the profile, and displays rhythmic visual stimulation on LEDs, a computer monitor, a TV monitor, or other suitable light output device, which is directed toward the eye.

The result of the inventive method is therefore that the system senses electrical or magnetic fields in the brain, amplifies the brain signal, and filters the signal to identify specific neural frequencies. In some embodiments, the system then collects output from the user's brain based on the brain's receipt of the visual and audio stimulation, and returns this feedback to the ES to further optimize the visual and audio stimulation.

The present inventors have found that the disclosed system and methods can entrain and drive oscillatory neural activity that is involved in learning, memory, and cognition. By providing music as the sole auditory stimulus, plus visual stimulation in the delta, theta, and/or gamma frequency bands, the inventive system and methods can serve as a method for treating, preventing, protecting against or otherwise affecting Alzheimer's Disease and dementia.

### STATEMENT OF INDUSTRIAL APPLICABILITY

The instant invention is an inventive device and method for treating and ameliorating the symptoms of Alzheimer's Disease and dementia. By providing music as the sole auditory stimulus, plus visual stimulation in the delta, theta, and/or gamma frequency bands, the inventive system and methods can serve as a method for treating, preventing, protecting against or otherwise affecting Alzheimer's Disease and dementia. Because Alzheimer's Disease and dementia are conditions which impact a large and growing portion of the world population, the innovative device and method have immediate applicability for treatment of current patients and those who have not yet experienced a full-scale onset of symptoms.

## Claims

1. A neural stimulation system, the system comprising:
an auditory analysis system configured to receive an acoustic input;
an entrainment simulator operatively connected to said auditory analysis system, said entrainment simulator configured to simulate neural entrainment to the acoustic input;
an oscillation selection module operatively connected to said entrainment simulator, said oscillation selection module configured to select one or more oscillations in one or more frequency ranges for stimulation;
a brain rhythm stimulator operatively connected to said oscillation selection module, said brain rhythm stimulator configured to generate a stimulation pattern according to the one or more oscillations, the stimulation pattern synchronized with the acoustic input; and
an output device (100) operatively connected to said brain rhythm stimulator, said output device configured to provide the stimulation pattern.

2. The neural stimulation system according to claim 1, wherein said auditory analysis system comprises a filtering module having means to filter the acoustic input comprising an incoming audio signal, an onset detection module having means to detect an onset of acoustic events in said signal, and a gain control module having means to adjust a gain of said signal, optionally wherein said auditory analysis system provides output to said entrainment simulator in the form of an onset signal.

3. The neural stimulation system according to claim 1 or claim 2, wherein
said entrainment simulator includes means to predict the frequencies, phases and amplitudes of a human neural response to music; and wherein
said entrainment simulator provides output to said oscillation selection module in the form of one or more network states;
optionally wherein said one or more frequency ranges include delta, theta, and gamma frequency bands.

4. The neural stimulation system according to claim 1, claim 2 or claim 3, wherein
said oscillation selection module includes means to select a most prominent oscillation in the one or more frequency ranges, and to couple a gamma frequency to a beat and rhythmic structure of music through theta-gamma and delta-theta PAC; and wherein
said oscillation selection module provides output to said brain rhythm stimulator in the form of said one or more oscillations.

5. The neural stimulation system according to any one of claims 1 to 4, wherein said brain rhythm stimulator comprises a light pattern buffer, a light generation module, a light adjustment module, and a filtering component, optionally wherein said output device (100) includes a device selected from the group comprising LED lights, a computer monitor, a TV monitor, goggles, a virtual reality headset, augmented reality glasses, and smart glasses.

6. The neural stimulation system according to claim 5, wherein said output device (100) is a visual stimulation ring, and wherein said visual stimulation ring comprises a cylindrical body having one or more LED lights (102) disposed around a circumferentially interior surface thereof, said one or more LED lights (102) being operatively connected to said brain rhythm stimulator, optionally wherein said brain rhythm stimulator provides output to said output device in the form of a control signal, said control signal containing instructions to cause said output device to display a rhythmic light pattern corresponding to said one or more oscillations selected by said oscillation selection module.

7. The neural stimulation system according to any one of claims 1 to 6, wherein said output device (100) comprises one or more stimulation units for generating tactile, vibratory stimuli, thermal stimuli, electrical transcutaneous stimuli, or electromagnetic fields and/or electrical currents to deliver transcranial stimulation, optionally wherein said output device is selected from the group comprising: a wearable device, gloves, a smart watch, a mobile device, or an array of electromagnets or electrodes.

8. The neural stimulation system according to any one of claims 1 to 7, further comprising a brain oscillation monitor operatively connected to said entrainment simulator, and with one or more means to sense electrical or magnetic fields in the brain, optionally wherein said one or more means to sense electrical or magnetic fields in the brain includes one or more devices selected from the group comprising electroencephalogram (EEG), intracranial EEG (iEEG), electrocorticography (ECoG), or magnetoencephalography (MEG).

9. The neural stimulation system according to any one of claims 1 to 8, wherein said auditory analysis system includes one or more means to receive said auditory input, said one or more means selected from the group comprising a built-in audio playback system, an auxiliary audio input or a microphone, said one or more means to receive an auditory input operatively coupled to said auditory analysis system, optionally further comprising an auditory output means.

10. The neural stimulation system according to any one of claims 1 to 9, further comprising a profile manager comprising a processor or internet-enabled software application accessing non-transitory and/or random-access memory which stores data pertaining to one or more users, said data being selected from a list comprising: identifying information, stored information from previous therapies, a library of audio files, and/or one or more user preferences, optionally wherein said profile manager is operatively connected to said entrainment simulator, said auditory analysis system, and said brain rhythm stimulator.

11. A method for generating a stimulation pattern for use in neural stimulation using the system of any one of claims 1 to 10, the method comprising:
selecting, by the oscillation module, the one or more oscillations in one or more predetermined frequency ranges;
generating, by the brain rhythm stimulator, the stimulation pattern using said one or more oscillations; and
outputting, by the output device (100), the stimulation pattern on an output device;
optionally wherein said stimulation pattern includes a type of stimulation selected from the group comprising: light patterns, vibro-tactile patterns, or patterns of electrical or magnetic pulses.

12. A method for generating a stimulation pattern for use in neural stimulation using the system of any one of claims 1 to 10, the method comprising:
receiving an auditory input comprising an acoustic signal;
filtering said acoustic signal;
detecting the onset of one or more acoustic events contained within said acoustic signal;
simulating neural entrainment to said acoustic signal using an entrainment simulator;
generating one or more coupling parameters to couple said one or more oscillations;
using adaptive learning algorithms to adjust said one or more coupling parameters or intrinsic parameters;
selecting one or more oscillations in one or more predetermined frequency ranges for display;
generating a light pattern using said one or more oscillations; and
displaying said light pattern on a visual output device.

13. The method for generating a stimulation pattern for use in neural stimulation of claim 12, the method further comprising:
prior to said step of generating a light pattern, the step of reading a patient profile from a profile manager; and
wherein said step of generating a light pattern comprises selecting a light pattern based on said patient profile, and adjusting said light pattern based on said patient profile.

14. The method for generating a stimulation pattern for use in neural stimulation of claim 12 or clam 13, the method further comprising:
receiving an input from a brain oscillation monitor;
providing said input to one or more multi-frequency neural networks;
coupling said input using one or more coupling parameters; and
using adaptive learning algorithms to adjust said one or more parameters to optimize the frequency, amplitude and phase of one or more outgoing oscillation signals,
wherein said one or more outgoing oscillation signals are used to generate and display said light pattern on said visual output device,
optionally wherein said brain oscillation monitor comprises one or more means to sense electrical or magnetic fields in the brain selected from the group comprising electroencephalogram (EEG), intracranial EEG (iEEG), electrocorticography (ECoG), or magnetoencephalography (MEG).

15. The method for generating a stimulation pattern for use in neural stimulation of claim 12, claim 13 or claim 14, wherein said auditory input is a musical composition, and wherein said musical composition is played in a synchronized fashion with said light pattern.

## Patentansprüche

1. Nervenstimulationssystem, wobei das System Folgendes umfasst:
ein auditives Analysesystem, das dazu konfiguriert ist, eine akustische Eingabe zu empfangen;
einen Mitnahmesimulator, der betriebswirksam mit dem auditiven Analysesystem verbunden ist, wobei der Mitnahmesimulator dazu konfiguriert ist, eine Nervenmitnahme zu der akustischen Eingabe zu simulieren;
ein Schwingungsauswahlmodul, das betriebswirksam mit dem Mitnahmesimulator verbunden ist, wobei das Schwingungsauswahlmodul dazu konfiguriert ist, eine oder mehrere Schwingungen in einem oder mehreren Frequenzbereichen zur Stimulation auszuwählen;
einen Gehirnrhythmusstimulator, der betriebswirksam mit dem Schwingungsauswahlmodul verbunden ist, wobei der Gehirnrhythmusstimulator dazu konfiguriert ist, ein Stimulationsmuster gemäß der einen oder den mehreren Schwingungen zu erzeugen, wobei das Stimulationsmuster mit der akustischen Eingabe synchronisiert ist; und
eine Ausgabevorrichtung (100), die betriebswirksam mit dem Gehirnrhythmusstimulator verbunden ist, wobei die Ausgabevorrichtung dazu konfiguriert ist, das Stimulationsmuster bereitzustellen.

2. Nervenstimulationssystem nach Anspruch 1, wobei das auditive Analysesystem ein Filtermodul, das Mittel aufweist, um die akustische Eingabe, umfassend ein eingehendes Audiosignal, zu filtern, ein Anfangsdetektionsmodul, das Mittel aufweist, um einen Anfang akustischer Ereignisse in dem Signal zu detektieren, und ein Verstärkungssteuerungsmodul, das Mittel aufweist, um eine Verstärkung des Signals anzupassen, umfasst, gegebenenfalls wobei das auditive Analysesystem dem Mitnahmesimulator eine Ausgabe in Form eines Anfangssignals bereitstellt.

3. Nervenstimulationssystem nach Anspruch 1 oder Anspruch 2, wobei
der Mitnahmesimulator Mittel beinhaltet, um die Frequenzen, die Phasen und die Amplituden einer menschlichen Nervenreaktion auf Musik vorherzusagen; und wobei
der Mitnahmesimulator dem Schwingungsauswahlmodul eine Ausgabe in Form eines oder mehrerer Netzzustände bereitstellt; gegebenenfalls wobei der eine oder die mehreren Frequenzbereiche Delta-, Theta- und Gamma-Frequenzbänder beinhalten.

4. Nervenstimulationssystem nach Anspruch 1, Anspruch 2 oder Anspruch 3, wobei
das Schwingungsauswahlmodul Mittel beinhaltet, um eine auffälligste Schwingung in dem einen oder den mehreren Frequenzbändern auszuwählen und um eine Gamma-Frequenz mit einem Taktschlag und einer rhythmischen Struktur von Musik über eine Theta-Gamma- und Delta-Theta-PAC zu koppeln; und wobei das Schwingungsauswahlmodul dem Gehirnrhythmusstimulator eine Ausgabe in Form der einen oder der mehreren Schwingungen bereitstellt.

5. Nervenstimulationssystem nach einem der Ansprüche 1 bis 4, wobei der Gehirnrhythmusstimulator einen Lichtmusterpuffer, ein Lichterzeugungsmodul, ein Lichtanpassungsmodul und eine Filterkomponente umfasst, gegebenenfalls wobei die Ausgabevorrichtung (100) eine Vorrichtung beinhaltet, die aus der Gruppe ausgewählt ist, die LED-Lichter, einen Computermonitor, einen Fernsehmonitor, eine Schutzbrille, ein Headset virtueller Realität, eine Augmented-Reality-Brille und eine intelligente Brille umfasst.

6. Nervenstimulationssystem nach Anspruch 5, wobei die Ausgabevorrichtung (100) ein visueller Stimulationsring ist und wobei der visuelle Stimulationsring einen zylindrischen Körper umfasst, der ein oder mehrere LED-Lichter (102) aufweist, die um eine Umfangsinnenfläche davon angeordnet sind, wobei das eine oder die mehreren LED-Lichter (102) betriebswirksam mit dem Gehirnrhythmusstimulator verbunden sind, gegebenenfalls wobei der Gehirnrhythmusstimulator der Ausgabevorrichtung eine Ausgabe in Form eines Steuerungssignals bereitstellt, wobei das Steuerungssignal Anweisungen enthält, um die Ausgabevorrichtung dazu zu veranlassen, ein rhythmisches Lichtmuster anzuzeigen, das der einen oder den mehreren Schwingungen entspricht, die durch das Schwingungsauswahlmodul ausgewählt sind.

7. Nervenstimulationssystem nach einem der Ansprüche 1 bis 6, wobei die Ausgabevorrichtung (100) eine oder mehrere Stimulationseinheiten zum Erzeugen taktiler, vibrierender Reize, Wärmereize, elektrischer transkutaner Reize oder elektromagnetischer Felder und/oder elektrischer Ströme umfasst, um eine transkranielle Stimulation zu bieten, gegebenenfalls wobei die Ausgabevorrichtung aus der Gruppe ausgewählt ist, die Folgendes umfasst: eine am Körper tragbare Vorrichtung, Handschuhe, eine intelligente Uhr, eine mobile Vorrichtung oder ein Array von Elektromagneten oder Elektroden.

8. Nervenstimulationssystem nach einem der Ansprüche 1 bis 7, ferner umfassend einen Gehirnschwingungsmonitor, der betriebswirksam mit dem Mitnahmesimulator und mit einem oder mehreren Mitteln, um elektrische oder magnetische Felder in dem Gehirn zu erfassen, verbunden ist, gegebenenfalls wobei das eine oder die mehreren Mittel, um elektrische oder magnetische Felder in dem Gehirn zu erfassen, eine oder mehrere Vorrichtungen beinhalten, die aus der Gruppe ausgewählt sind, die ein Elektroenzephalogramm (EEG), ein intrakranielles EEG (iEEG), eine Elektrokortikographie (ECoG) oder eine Magnetenzephalographie (MEG) umfasst.

9. Nervenstimulationssystem nach einem der Ansprüche 1 bis 8, wobei das auditive Analysesystem ein oder mehrere Mittel, um die auditive Eingabe zu empfangen, beinhaltet, wobei das eine oder die mehreren Mittel aus der Gruppe ausgewählt sind, die ein eingebautes Audiowiedergabesystem, eine Hilfsaudioeingabe oder ein Mikrofon umfasst, wobei das eine oder die mehreren Mittel, um eine auditive Eingabe zu empfangen, betriebswirksam mit dem auditiven Analysesystem gekoppelt sind, gegebenenfalls ferner umfassend ein auditives Ausgabemittel.

10. Nervenstimulationssystem nach einem der Ansprüche 1 bis 9, ferner umfassend einen Profilverwalter, umfassend einen Prozessor oder eine internetfähige Softwareanwendung, die auf einen nichtflüchtigen und/oder Direktzugriffsspeicher zugreift, der Daten bezüglich eines oder mehrerer Benutzer speichert, wobei die Daten aus einer Liste ausgewählt sind, die Folgendes umfasst: Identifizierungsinformationen, gespeicherte Informationen aus vorigen Therapien, eine Bibliothek von Audiodateien und/oder eine oder mehrere Benutzerpräferenzen, gegebenenfalls wobei der Profilverwalter betriebswirksam mit dem Mitnahmesimulator, dem auditiven Analysesystem und dem Gehirnrhythmusstimulator verbunden ist.

11. Verfahren zum Erzeugen eines Stimulationsmusters zur Verwendung bei einer Nervenstimulation unter Verwendung des Systems nach einem der Ansprüche 1 bis 10, wobei das Verfahren Folgendes umfasst:
Auswählen, durch das Schwingungsmodul, der einen oder der mehreren Schwingungen in einem oder mehreren vorbestimmten Frequenzbereichen;
Erzeugen, durch den Gehirnrhythmusstimulator, des Stimulationsmusters unter Verwendung der einen oder der mehreren Schwingungen; und
Ausgeben, durch die Ausgabevorrichtung (100), des Stimulationsmusters auf einer Ausgabevorrichtung;
gegebenenfalls wobei das Stimulationsmuster eine Art von Stimulation beinhaltet, die aus der Gruppe ausgewählt ist, die Folgendes umfasst: Lichtmuster, vibrotaktile Muster oder Muster elektrischer oder magnetischer Impulse.

12. Verfahren zum Erzeugen eines Stimulationsmusters zur Verwendung bei einer Nervenstimulation unter Verwendung des Systems nach einem der Ansprüche 1 bis 10, wobei das Verfahren Folgendes umfasst:
Empfangen einer auditiven Eingabe, umfassend ein akustisches Signal;
Filtern des akustischen Signals;
Detektieren des Anfangs eines oder mehrerer akustischer Ereignisse, die innerhalb des akustischen Signals enthalten sind;
Simulieren einer Nervenmitnahme zu dem akustischen Signal unter Verwendung eines Mitnahmesimulators;
Erzeugen eines oder mehrerer Kopplungsparameter, um die eine oder die mehreren Schwingungen zu koppeln;
Verwenden adaptiver Lernalgorithmen, um den einen oder die mehreren Kopplungsparameter oder intrinsischen Parameter anzupassen;
Auswählen einer oder mehrerer Schwingungen in einem oder mehreren vorbestimmten Frequenzbereichen zur Anzeige;
Erzeugen eines Lichtmusters unter Verwendung der einen oder der mehreren Schwingungen; und
Anzeigen des Lichtmusters auf einer visuellen Ausgabevorrichtung.

13. Verfahren zum Erzeugen eines Stimulationsmusters zur Verwendung bei einer Nervenstimulation nach Anspruch 12, wobei das Verfahren ferner Folgendes umfasst:
vor dem Schritt des Erzeugens eines Lichtmusters, den Schritt zum Lesen eines Patientenprofils aus einem Profilverwalter; und
wobei der Schritt des Erzeugens eines Lichtmusters Auswählen eines Lichtmusters basierend auf dem Patientenprofil und
Anpassen des Lichtmusters basierend auf dem Patientenprofil umfasst.

14. Verfahren zum Erzeugen eines Stimulationsmusters zur Verwendung bei einer Nervenstimulation nach Anspruch 12 oder Sandklaffmuschel 13, wobei das Verfahren ferner Folgendes umfasst:
Empfangen einer Eingabe von einem Gehirnschwingungsmonitor;
Bereitstellen der Eingabe an ein oder mehrere Mehrfrequenznervennetze;
Koppeln der Eingabe unter Verwendung eines oder mehrerer Kopplungsparameter; und
Verwenden adaptiver Lernalgorithmen, um den einen oder die mehreren Parameter anzupassen, um die Frequenz, die Amplitude und die Phase eines oder mehrerer ausgehender Schwingungssignale zu optimieren,
wobei das eine oder die mehreren ausgehenden Schwingungssignale dazu verwendet werden, das Lichtmuster zu erzeugen und auf der visuellen Ausgabevorrichtung anzuzeigen,
gegebenenfalls wobei der Gehirnschwingungsmonitor ein oder mehrere Mittel, um elektrische oder magnetische Felder in dem Gehirn zu erfassen, umfasst, die aus der Gruppe ausgewählt sind, die ein Elektroenzephalogramm (EEG), ein intrakranielles EEG (iEEG), eine Elektrokortikographie (ECoG) oder eine Magnetenzephalographie (MEG) umfasst.

15. Verfahren zum Erzeugen eines Stimulationsmusters zur Verwendung bei einer Nervenstimulation nach Anspruch 12, Anspruch 13 oder Anspruch 14, wobei die auditive Eingabe eine musikalische Komposition ist und wobei die musikalische Komposition in einer synchronisierten Weise mit dem Lichtmuster wiedergegeben wird.

## Revendications

1. Système de stimulation neurale, le système comprenant :
un système d'analyse auditive configuré pour recevoir une entrée acoustique ;
un simulateur d'entraînement relié de manière opérationnelle audit système d'analyse auditive, ledit simulateur d'entraînement étant configuré pour simuler un entraînement neural à l'entrée acoustique ;
un module de sélection d'oscillation relié de manière opérationnelle audit simulateur d'entraînement, ledit module de sélection d'oscillation étant configuré pour sélectionner une ou plusieurs oscillations dans une ou plusieurs plages de fréquences pour la stimulation ;
un graphique de rythme cérébral relié de manière opérationnelle audit module de sélection d'oscillation, ledit stimulateur de rythme cérébral étant configuré pour générer un motif de stimulation selon les une ou plusieurs oscillations, le motif de stimulation étant synchronisé avec l'entrée acoustique ; et
un dispositif de sortie (100) relié de manière opérationnelle audit stimulateur de rythme cérébral, ledit dispositif de sortie étant configuré pour fournir le motif de stimulation.

2. Système de stimulation neurale selon la revendication 1, dans lequel ledit système d'analyse auditive comprend un module de filtrage ayant des moyens pour filtrer l'entrée acoustique comprenant un signal audio entrant, un module de détection d'attaque ayant des moyens pour détecter une attaque d'événements acoustiques dans ledit signal, et un module de commande de gain ayant des moyens pour ajuster un gain dudit signal, éventuellement dans lequel ledit système d'analyse auditive fournit une sortie audit simulateur d'entraînement sous la forme d'un signal d'attaque.

3. Système de stimulation neurale selon la revendication 1 ou la revendication 2, dans lequel
ledit simulateur d'entraînement inclut des moyens pour prédire les fréquences, phases et amplitudes d'une réponse neurale humaine à la musique ; et dans lequel
ledit simulateur d'entraînement fournit une sortie audit module de sélection d'oscillation sous la forme d'un ou plusieurs états de réseau ;
éventuellement dans lequel les dits une ou plusieurs plages de fréquences incluent des bandes de fréquences delta, thêta et gamma.

4. Système de stimulation neurale selon la revendication 1, la revendication 2 ou la revendication 3, dans lequel ledit module de sélection d'oscillation inclut des moyens pour sélectionner une oscillation la plus proéminente dans les une ou plusieurs plages de fréquences, et pour coupler une fréquence gamma à un temps et à une structure rythmique de musique par l'intermédiaire d'un PAC thêta-gamma et delta-thêta ; et dans lequel
ledit module de sélection d'oscillation fournit une sortie audit stimulateur de rythme cérébral sous la forme des dits une ou plusieurs oscillations.

5. Système de stimulation neurale selon l'une quelconque des revendications 1 à 4, dans lequel ledit stimulateur de rythme cérébral comprend un tampon de motif lumineux, un module de génération de lumière, un module d'ajustement de lumière et un composant de filtrage, éventuellement dans lequel ledit dispositif de sortie (100) inclut un dispositif sélectionné dans le groupe comprenant des lumières DEL, un moniteur d'ordinateur, un moniteur de télévision, des lunettes de protection, un casque de réalité virtuelle, des lunettes de réalité augmentée et des lunettes intelligentes.

6. Système de stimulation neurale selon la revendication 5, dans lequel ledit dispositif de sortie (100) est un anneau de stimulation visuelle, et dans lequel ledit anneau de stimulation visuelle comprend un corps cylindrique ayant une ou plusieurs lumières DEL (102) disposées autour d'une surface intérieure circonférentielle de celui-ci, les dits une ou plusieurs lumières DEL (102) étant reliées de manière opérationnelle audit stimulateur de rythme cérébral, éventuellement dans lequel ledit stimulateur de rythme cérébral fournit une sortie audit dispositif de sortie sous la forme d'un signal de commande, ledit signal de commande contenant des instructions pour amener ledit dispositif de sortie à afficher un motif lumineux rythmique correspondant aux dits une ou plusieurs oscillations sélectionnées par ledit module de sélection d'oscillation.

7. Système de stimulation neurale selon l'une quelconque des revendications 1 à 6, dans lequel ledit dispositif de sortie (100) comprend une ou plusieurs unités de stimulation pour générer des stimuli tactiles, vibratoires, des stimuli thermiques, des stimuli transcutanés électriques, ou des champs électromagnétiques et/ou des courants électriques pour délivrer une stimulation transcranienne, éventuellement dans lequel ledit dispositif de sortie est sélectionné dans le groupe comprenant : un dispositif portable, des gants, une montre intelligente, un dispositif mobile, ou un réseau d'électroaimants ou d'électrodes.

8. Système de stimulation neurale selon l'une quelconque des revendications 1 à 7, comprenant en outre un moniteur d'oscillation cérébrale relié de manière opérationnelle audit simulateur d'entraînement, et avec un ou plusieurs moyens pour détecter des champs électriques ou magnétiques dans le cerveau, éventuellement dans lequel les dits un ou plusieurs moyens pour détecter des champs électriques ou magnétiques dans le cerveau incluent un ou plusieurs dispositifs sélectionnés dans le groupe comprenant l'électroencéphalogramme (EEG), l'EEG intracrânien (iEEG), l'électrocorticographie (ECoG) ou la magnétoencéphalographie (MEG).

9. Système de stimulation neurale selon l'une quelconque des revendications 1 à 8, dans lequel ledit système d'analyse auditive inclut un ou plusieurs moyens pour recevoir ladite entrée auditive, les dits un ou plusieurs moyens étant sélectionnés dans le groupe comprenant un système de lecture audio intégré, une entrée audio auxiliaire ou un microphone, les dits un ou plusieurs moyens pour recevoir une entrée auditive étant couplés de manière opérationnelle audit système d'analyse auditive, éventuellement comprenant en outre un moyen de sortie auditive.

10. Système de stimulation neurale selon l'une quelconque des revendications 1 à 9, comprenant en outre un gestionnaire de profil comprenant un processeur ou une application logicielle compatible Internet accédant à une mémoire non transitoire et/ou à accès aléatoire qui stocke des données relatives à un ou plusieurs utilisateurs, les dits données étant sélectionnées dans une liste comprenant : des informations d'identification, des informations stockées provenant de thérapies précédentes, une bibliothèque de fichiers audio et/ou une ou plusieurs préférences d'utilisateur, éventuellement dans lequel ledit gestionnaire de profil est relié de manière opérationnelle audit simulateur d'entraînement, audit système d'analyse auditive et audit stimulateur de rythme cérébral.

11. Procédé pour générer un motif de stimulation pour une utilisation dans une stimulation neurale utilisant le système de l'une quelconque des revendications 1 à 10, le procédé comprenant :
la sélection, par le module d'oscillation, des une ou plusieurs oscillations dans une ou plusieurs plages de fréquences prédéterminées ;
la génération, par le stimulateur de rythme cérébral, du motif de stimulation en utilisant les dits une ou plusieurs oscillations ; et
la sortie, par le dispositif de sortie (100), du motif de stimulation sur un dispositif de sortie ;
éventuellement dans lequel ledit motif de stimulation inclut un type de stimulation sélectionné dans le groupe comprenant :
des motifs lumineux, des motifs vibro-tactiles ou des motifs d'impulsions électriques ou magnétiques.

12. Procédé pour générer un motif de stimulation pour une utilisation dans une stimulation neurale utilisant le système de l'une quelconque des revendications 1 à 10, le procédé comprenant les étapes consistant à :
recevoir une entrée auditive comprenant un signal acoustique ; filtrer ledit signal acoustique ;
détecter l'attaque d'un ou plusieurs événements acoustiques contenus dans ledit signal acoustique ;
simuler un entraînement neural audit signal acoustique en utilisant un simulateur d'entraînement ;
générer un ou plusieurs paramètres de couplage pour coupler les dits une ou plusieurs oscillations ;
utiliser des algorithmes d'apprentissage adaptatif pour ajuster les dits un ou plusieurs paramètres de couplage ou paramètres intrinsèques ;
sélectionner une ou plusieurs oscillations dans une ou plusieurs plages de fréquences prédéterminées pour l'affichage ;
générer un motif lumineux en utilisant les dits une ou plusieurs oscillations ; et afficher ledit motif lumineux sur un dispositif de sortie visuelle.

13. Procédé pour générer un motif de stimulation pour une utilisation dans une stimulation neurale selon la revendication 12, le procédé comprenant en outre :
avant ladite étape consistant à générer un motif lumineux, l'étape consistant à lire un profil de patient à partir d'un gestionnaire de profil ; et
dans lequel ladite étape consistant à générer un motif lumineux comprend la sélection d'un motif lumineux sur la base dudit profil de patient, et l'ajustement dudit motif lumineux sur la base dudit profil de patient.

14. Procédé pour générer un motif de stimulation pour une utilisation dans une stimulation neurale selon la revendication 12 ou la revendication 13, le procédé comprenant en outre :
la réception d'une entrée provenant d'un moniteur d'oscillation cérébrale ;
la fourniture de ladite entrée à un ou plusieurs réseaux neuraux multifréquences ;
le couplage de ladite entrée en utilisant un ou plusieurs paramètres de couplage ; et
l'utilisation d'algorithmes d'apprentissage adaptatif pour réaliser l'ajustement des dits un ou plusieurs paramètres afin d'optimiser la fréquence, l'amplitude et la phase d'un ou plusieurs signaux d'oscillation sortants,
dans lequel les dits un ou plusieurs signaux d'oscillation sortants sont utilisés pour générer et afficher ledit motif lumineux sur ledit dispositif de sortie visuelle,
éventuellement dans lequel ledit moniteur d'oscillation cérébrale comprend un ou plusieurs moyens pour détecter des champs électriques ou magnétiques dans le cerveau sélectionnés dans le groupe comprenant l'électroencéphalogramme (EEG), l'EEG intracrânien (iEEG), l'électrocorticographie (ECoG) ou la magnétoencéphalographie (MEG).

15. Procédé pour générer un motif de stimulation pour une utilisation dans une stimulation neurale selon la revendication 12, la revendication 13 ou la revendication 14, dans lequel ladite entrée auditive est une composition musicale, et dans lequel ladite composition musicale est lue de manière synchronisée avec ledit motif lumineux.
